(19) Europäisches Patentamt
European Patent Office
Office européen des brevets

(11) **EP 2 123 275 A1**

(12) **EUROPEAN PATENT APPLICATION**
published in accordance with Art. 153(4) EPC

(43) Date of publication:
**25.11.2009 Bulletin 2009/48**

(21) Application number: **08722190.9**

(22) Date of filing: **14.03.2008**

(51) Int Cl.:
*A61K 31/485* (2006.01)      *A61K 9/22* (2006.01)
*A61K 9/70* (2006.01)      *A61K 47/26* (2006.01)
*A61K 47/32* (2006.01)      *A61P 25/04* (2006.01)

(86) International application number:
**PCT/JP2008/054794**

(87) International publication number:
**WO 2008/111674 (18.09.2008 Gazette 2008/38)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.03.2007   JP 2007066807**

(71) Applicants:
- **Toyo Boseki Kabushiki Kaisha**
  **Osaka-shi, Osaka 530-8230 (JP)**
- **Fuso Pharmaceutical Industries, Inc.**
  **Osaka-shi**
  **Osaka 541-0045 (JP)**

(72) Inventors:
- **MUKUNOKI, Fuminori**
  **Ohtsu-shi**
  **Shiga 520-0292 (JP)**
- **MIYASAKA, Tadayo**
  **Osaka-shi**
  **Osaka 530-8230 (JP)**

(74) Representative: **Vossius & Partner**
  **Siebertstraße 4**
  **81675 München (DE)**

(54) **PROCESS FOR PRODUCTION OF BUPRENORPHINE PHARMACEUTICAL PREPARATION TO BE APPLIED TO MOUTH MUCOSA**

(57) The objective of the present invention is to provide a process for producing a buprenorphine pharmaceutical preparation to be applied to mouth mucosa. The process according to the present invention for producing a buprenorphine pharmaceutical preparation to be applied to mouth mucosa includes steps of: preparing a granule for an immediate-release layer containing a buprenorphine hydrochloride crystal having 90% cumulative diameter of 200 $\mu$m or less; preparing a granule for a sustained-release layer containing a buprenorphine hydrochloride crystal having 90% cumulative diameter of 250 $\mu$m or less; and tabletting the granule for the immediate-release layer and the granule for the sustained-release layer into a two-layer tablet.

EP 2 123 275 A1

**Description**

TECHNICAL FIELD

The present invention relates to a process for the production of a buprenorphine pharmaceutical preparation to be applied to mouth mucosa.

BACKGROUND ART

Buprenorphine hydrochloride is a nonnarcotic analgesic agent and is sold in the form of an injection and a suppository in Japan. Further, buprenorphine hydrochloride is sold in the form of a sublingual tablet in some countries other than Japan.

However, when the above pharmaceutical preparations are administered, the effect is transient since the blood level of buprenorphine falls immediately after rising sharply. Thus, high frequency of administration is needed for chronic pain, and hence a problem of an adverse effect arises. In addition, the sublingual tablet has a problem that the absorption efficiency of buprenorphine is poor. Further, there are some examples that buprenorphine is used as external preparation; but the external preparation is not practical since the cutaneous absorption of buprenorphine is extremely poor.

The researchers in the applicant of the present application developed a pharmaceutical preparation of buprenorphine hydrochloride to be applied to mouth mucosa mainly for the purpose of suppressing cancer pain. The pharmaceutical preparation is disclosed in JP8-291070A. In the Example described in the publication, a two-layer pharmaceutical preparation consisting of a sustained-release layer and an immediate-release layer is produced in such a manner that untreated buprenorphine hydrochloride is homogenously mixed with other ingredients and then tabletted. However, the amount of the medicinal ingredient is nonuniform between pharmaceutical preparations produced in such a manner. Therefore, the researchers in the applicant of the present application examined a technology that buprenorphine hydrochloride is dissolved in purified water and the solution is used for obtaining uniform pharmaceutical preparations.

Further, JP2001-506640T discloses a buprenorphine pharmaceutical preparation to be applied in the oral cavity. The preparation is produced by using a buprenorphine solution.

However, the stability of buprenorphine in a pharmaceutical preparation produced by using a buprenorphine hydrochloride solution is relatively poor, and there is a problem that decomposed substances are generated in such a pharmaceutical preparation.

SUMMARY OF THE INVENTION

Conventionally, as described above, a buprenorphine hydrochloride solution is used when a pharmaceutical preparation is produced, in order to prevent the amount of buprenorphine hydrochloride as a medicinal ingredient from being nonuniform between pharmaceutical preparations. However, the stability of buprenorphine is relatively poor in such a pharmaceutical preparation, and it is found that there is a problem that decomposed substances are generated in the pharmaceutical preparation.

Under the above situation, the objective of the present invention is to provide a process for producing a pharmaceutical preparation to be applied to mouth mucosa, which have excellent stability and in which the amount of buprenorphine therein is uniform.

The present inventors thoroughly studied conditions for producing a pharmaceutical preparation to be applied to mouth mucosa, which have excellent stability and have uniform amount of buprenorphine therein. As a result, the present inventors found that the above problems can be solved by appropriately controlling the particle diameter of a buprenorphine hydrochloride crystal as a raw material and using the crystal; and completed the present invention.

The process according to the present invention for producing a buprenorphine pharmaceutical preparation to be applied to mouth mucosa is characterized in comprising steps of: preparing a granule for an immediate-release layer containing a buprenorphine hydrochloride crystal having 90% cumulative diameter of 200 μm or less; preparing a granule for a sustained-release layer containing a buprenorphine hydrochloride crystal having 90% cumulative diameter of 250 μm or less; and tabletting the granule for the immediate-release layer and the granule for the sustained-release layer into a two-layer tablet.

BRIEF DESCRIPTION OF THE DRAWINGS

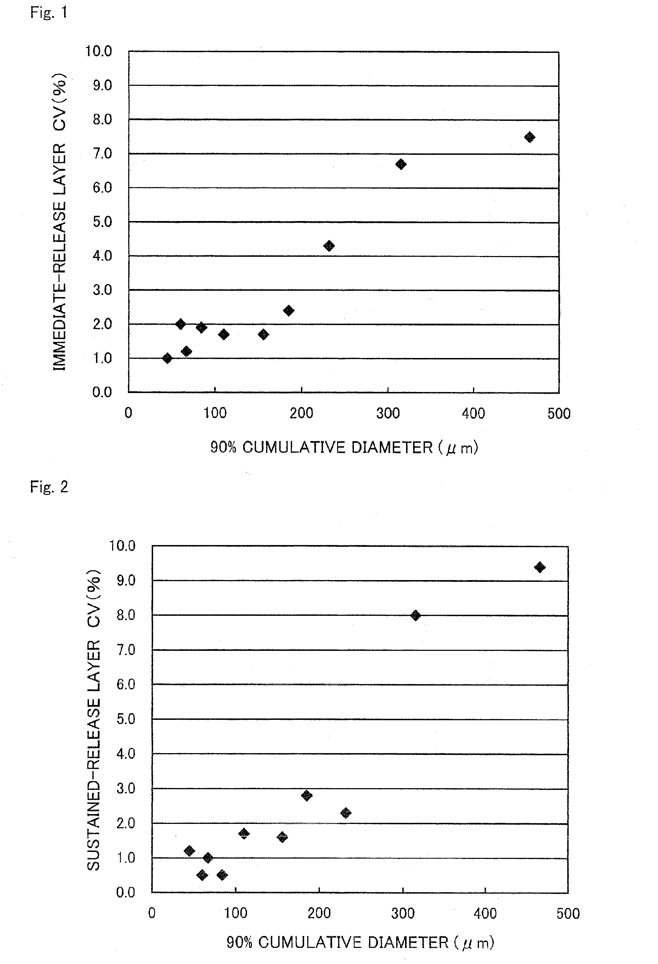

Fig. 1 is a graph showing the relation in an immediate-release layer of a buprenorphine hydrochloride two-layer tablet between the 90% cumulative diameter of a buprenorphine hydrochloride crystal and the relative standard deviation of the buprenorphine hydrochloride amount in a granule for the immediate-release layer.

FIG. 2 is a graph showing a relation in a sustained-release layer of the buprenorphine hydrochloride two-layer tablet between the 90% cumulative diameter of a buprenorphine hydrochloride crystal and the relative standard deviation of the buprenorphine hydrochloride amount in a granule for the sustained-release layer.

BEST MODE FOR CARRYING OUT THE INVENTION

The process according to the present invention for producing a buprenorphine pharmaceutical preparation to be applied to mouth mucosa is characterized in comprising steps of: preparing a granule for an immediate-release layer containing a buprenorphine hydrochloride crystal having 90% cumulative diameter of 200 $\mu$m or less; preparing a granule for a sustained-release layer containing a buprenorphine hydrochloride crystal having 90% cumulative diameter of 250 $\mu$m or less; and tabletting the granule for the immediate-release layer and the granule for the sustained-release layer into a two-layer tablet. The each step is described in the following.

(1) Step for preparing a granule for an immediate-release layer

In the present invention, first, a buprenorphine hydrochloride crystal is disintegrated such that the 90% cumulative diameter thereof becomes 200 $\mu$m or less. In conventional production of a buprenorphine pharmaceutical preparation to be applied to mouth mucosa, a solution obtained by dissolving buprenorphine hydrochloride in purified water is used. However, it is found that the preservation stability is deteriorated when the solution is used. On the other hand, when an untreated buprenorphine hydrochloride crystal is used instead of the solution, the buprenorphine hydrochloride amount in an immediate-release layer is reduced, though not to the extent as in a sustained-release layer. In the present invention, a buprenorphine hydrochloride crystal having 90% cumulative diameter of 200 $\mu$m or less is used.

The reason why the 90% cumulative diameter of the buprenorphine hydrochloride crystal is determined to be 200 $\mu$m or less is to prevent a reduction and nonuniformity of the buprenorphine hydrochloride amount during production of a pharmaceutical preparation, as described above. The reason why the 90% cumulative diameter is used as reference is that a crystal having extremely large diameter is possibly contained depending on a particle size distribution when only 50% cumulative diameter which is more commonly used is determined, and such a large crystal can cause reduction or segregation of buprenorphine hydrochloride amount. The 90% cumulative diameter is more preferably 160 $\mu$m or less, even more preferably 100 $\mu$m or less, and particularly preferably 90 $\mu$m or less. Further, the 50% cumulative diameter is preferably 100 $\mu$m or less, and more preferably 60 $\mu$m or less. On the other hand, the 50% cumulative diameter is preferably 10 $\mu$m or more, since there is the possibility of reaggregation due to the pulverization when the particle diameter is excessively reduced.

The 90% cumulative diameter and the 50% cumulative diameter can be measured by an ordinary method. Specifically, for example, a buprenorphine hydrochloride crystal is dispersed in a McIlvaine buffer solution (pH 9.0) containing about 0.1% of a surfactant such as Triton X-100, and the volume-based particle size distribution of the buprenorphine hydrochloride crystal is measured by a particle size distribution measuring apparatus. From the obtained measurement result, the particle diameter when the cumulative volume from smaller particles is 50% is regarded as a 50% cumulative diameter, and the particle diameter when the cumulative volume is 90% is regarded as a 90% cumulative diameter, on the assumption that the total volume is regarded as 100%.

The method for controlling the particle diameter is not particularly limited, and a general grinding machine such as a hammer mill may be used, and disintegration and a particle size distribution measurement may be repeated until the particle diameter becomes appropriate. Further, the particle diameter may be adjusted by sieving.

The buprenorphine hydrochloride crystal whose particle size is controlled is mixed with other ingredients. Formulation ingredients may be selected as appropriate; but the immediate-release layer contains ingredients used as a diluent or a disintegrator in a relatively large amount, since the immediate-release layer has to disintegrate relatively fast to release buprenorphine hydrochloride. Preferably, D-mannitol and/or talc are contained.

The buprenorphine hydrochloride crystal and the other ingredients are mixed in a dry condition without using water. If water is used, there is the possibility that buprenorphine hydrochloride becomes unstable. The mixing may be carried out by using a general mixer. The particle size of the mixture is preferably adjusted by sieving or the like for suppressing adhesion between the particles and the like.

Next, a granule is prepared by using the mixture, a polyvinylpyrrolidone solution as a binding agent and the like. On the occasion, a fluid layer granulator generally used in the pharmaceutical preparation field can be used. General conditions for preparing granule can be used, and the detailed condition may be set as appropriate.

Other ingredient may be added to the mixture including the buprenorphine hydrochloride crystal. The example of the other ingredient includes lubricants such as pharmaceutically acceptable magnesium stearate, a pharmaceutically acceptable talc and the like. In addition, known pharmaceutical ingredients such as a pharmaceutically acceptable flavoring substance, a pharmaceutically acceptable coloring agent and the like may be blended.

After the granulation, the granule is sufficiently dried. The drying conditions such as temperature, time and the like are not particularly limited as long as each ingredient is not changed in quality. Further, the particle size of the obtained granule is preferably adjusted by sieving or the like.

(2) Step for preparing a granule for a sustained-release layer

A buprenorphine hydrochloride crystal to be added for a sustained-release layer is disintegrated in advance such that the 90% cumulative diameter thereof becomes 250 μm or less. According to the finding by the present inventors, when a buprenorphine hydrochloride solution is not used during production of a pharmaceutical preparation, a reduction of the buprenorphine hydrochloride amount in the sustained-release is particularly marked. In the present invention a reduction and nonuniformity of the active ingredient amount during production of a pharmaceutical preparation are suppressed while the preservation stability of the active ingredient is maintained by using a buprenorphine hydrochloride crystal having 90% cumulative diameter of 250 μm or less.

Similarly to the buprenorphine hydrochloride crystal for the immediate-release layer, it is preferable that the 90% cumulative diameter of the buprenorphine hydrochloride crystal for the sustained-release layer is smaller. Thus, the 90% cumulative diameter is more preferably 160 μm or less, and even more preferably 100 μm or less. Further, an appropriate value of the 50% cumulative diameter is the same as that for the immediate-release layer. In addition, the same disintegration means as that in the case of the immediate-release layer can be used.

The buprenorphine hydrochloride crystal whose particle size is adjusted is mixed with other ingredients. Formulation ingredients may be selected as appropriate, and the sustained-release layer preferably contains polyvinylpyrrolidone or a pharmaceutically acceptable salt thereof, polyacrylic acid or a pharmaceutically acceptable salt thereof, and sodium hydrogen carbonate in addition to buprenorphine hydrochloride, since the sustained-release layer has to disintegrate gradually to release buprenorphine hydrochloride in a sustained manner.

By causing polyvinylpyrrolidone or the pharmaceutically acceptable salt thereof and polyacrylic acid or the pharmaceutically acceptable salt thereof to coexist in the sustained-release layer of the pharmaceutical preparation of the present invention, a sustained-release property is given to the sustained-release layer. In other words, by causing polyvinylpyrrolidone having a binding ability and polyacrylic acid having an adhesive ability to coexist in the sustained-release layer, an appropriate sustained-release property is given to the sustained-release layer. The ratio of both may be adjusted as appropriate, and the ratio of the amount of polyvinylpyrrolidone or the pharmaceutically acceptable salt thereof relative to the total amount of polyvinylpyrrolidone or the pharmaceutically acceptable salt thereof and polyacrylic acid or the pharmaceutically acceptable salt thereof is preferably 5% or more by mass and 95% or less by mass. In case that the ratio is 5% or less by mass, there is the possibility that feeling of a foreign substance occurs due to the excessively great expansibility of the tablet when the tablet is applied in the oral cavity. On the other hand, when the ratio exceeds 95% by mass, there is the possibility that the adhesion of the tablet becomes weak.

Further, the sustained-release layer of the tablet of the present invention preferably contains sodium hydrogen carbonate. Buprenorphine hydrochloride that is an active ingredient of the tablet of the present invention is acidic, and the swellability of polyacrylic acid may be reduced under an acidic condition. Thus, sodium hydrogen carbonate is preferably contained as a pH adjuster for neutralizing the sustained-release layer. The amount of sodium hydrogen carbonate in the sustained-release layer is preferably in a ratio of 7.0% or more by mass and 7.5% or less by mass relative to polyacrylic acid or the pharmaceutically acceptable salt thereof. When the ratio is 7.0% or more by mass, sufficient swellability of polyacrylic acid can be ensured, and thus buprenorphine hydrochloride can be released more assuredly. On the other hand, when the ratio exceeds 7.5% by mass, the swelling of polyacrylic acid can become excessive, resulting in that buprenorphine hydrochloride is released excessively fast.

The example of the pharmaceutically acceptable salts of polyvinylpyrrolidone and polyacrylic acid includes alkali metal salts such as a sodium salt, a potassium salt and the like; earth metal salts such as a calcium salt, a magnesium salt and the like; and ammonium salts.

In addition, the sustained-release layer may contain conventional formulation ingredients such as a lubricant, a binding agent, a flavoring substance, a coloring agent and the like.

The mixing of the formulation ingredients may be carried out by using a general mixer. After the mixing, the particle size of the mixture is preferably adjusted. As the particle size adjustment, disintegration and size-regulation are preferably carried out. By such disintegration and size-regulation in the step for preparing a granule for the sustained-release layer, a reduction and nonuniformity of the buprenorphine hydrochloride amount in the sustained-release during production of a pharmaceutical preparation can be suppressed more. Such disintegration and size-regulation are not simple sieving, and are to disintegrate the mixture by using a power mill or the like and to control the size of the mixture while the adhesion between the particles is suppressed.

A granule for the sustained-release layer is prepared from the above mixture. The preparation of the granule may be carried out according to an ordinary method. For example, the above mixture may be merely compressed. The size of the obtained compression molded product is preferably adjusted again, and more preferably subjected to disintegration and size-regulation as the above. In the size-regulation, sieving may be carried out in combination.

(3) Tabletting step

Next, the above granule for the layer immediate-release and the above granule for the sustained-release layer are tabletted into a two-layer tablet. A tabletting machine is not particularly limited as long as the machine is capable of producing a two-layer tablet. The tabletting condition may be adjusted while the weights of the sustained-release layer and the immediate-release layer, the hardness of the tablet, the thickness of the tablet and the like are measured as appropriate from a tablet sample.

During production of a tablet, variation in the active ingredient amount per tablet needs to be suppressed. Further, of course, the preservation stability is extremely important. However, conventional processes for producing a buprenorphine pharmaceutical preparation to be applied to mouth mucosa do not provide desired preservation stability of buprenorphine hydrochloride and desired suppression of variation in the buprenorphine hydrochloride amount.

On the other hand, according to the method of the present invention, a tablet can be produced so as to achieve both of desired preservation stability of buprenorphine hydrochloride and desired suppression of variation of the buprenorphine hydrochloride amount.

In the buprenorphine hydrochloride preparation produced by the process of the present invention, the reduction of the buprenorphine hydrochloride amount during production is suppressed and the preservation stability of buprenorphine hydrochloride is excellent. For example, even when the preparation is stored for 6 months in a severe environment of a temperature of 40°C and a relative humidity of 75%, the amount of buprenorphine derivatives is suppressed.

When the preparation of the present invention is applied to mouth mucosa, the immediate-release layer disintegrates first to release buprenorphine hydrochloride, and the sustained-release layer gradually disintegrates to release buprenorphine hydrochloride slowly. Thus, the preparation of the present invention does not provide a sharp rise of the blood level of buprenorphine as compared to an injection and the like, and is less likely to cause an adverse effect, and has excellent persistence of the medicinal effect. Therefore, the preparation of the present invention is effective for the therapy of postherpetic neuralgia and the like in which chronic pain continues and the painful part cannot be clearly recognized.

EXAMPLES

Hereinafter, the present invention is described in more detail with reference to the Examples; but, of course, the present invention is not limited to the Examples and can be carried out in such a range as to be adapted to the purport of the description in the specification; and any of such modifications are included in the scope of the present invention.

Comparative Example 1

(1) Preparation of a granule for an immediate-release layer

While purified water (431.2 g) was stirred, polyvinylpyrrolidone (manufactured by BASF, product name: PVP K30, 27.4 g) was added thereto. After it was visually confirmed that the solution was in a homogenous state, buprenorphine hydrochloride (2.156 g) was added into the solution. After it was visually confirmed that the solution was in a homogenous state, Food Blue No. 1 (0.03 g) was added and dissolved in the solution. The above solution and D-mannitol (723 g) were put in a fluid layer granulator (manufactured by Freund Corporation, Model: FLO-5B), and granulation was carried out using purified water (50 g) as a rinse. Subsequently, the obtained granule was dried by using the granulator under the conditions of an intake air temperature of $70 \pm 5$°C and an airflow rate of $2.0 \pm 0.5$ m$^3$/min until the exhaust air temperature became 40°C. To 100 parts by mass of the obtained granule, 1 parts by mass of sodium stearate and 0.56 parts by mass of talc were added, and mixed by using a rocking mixer (manufactured by Aichi Electric Co., Ltd., Model: RM-10) at $60 \pm 3$ rpm for 3 minutes, to prepare a granule for an immediate-release layer.

(2) Preparation of a granule for a sustained-release layer

Apart from the above, while purified water (862.4 g) was stirred, polyvinylpyrrolidone (manufactured by BASF, product name: PVP K90, 8.624 g) was added thereto. After it was visually confirmed that the solution was in a homogenous state, buprenorphine hydrochloride (4.312 g) was added and dissolved in the solution. The solution and polyvinylpyrrolidone (manufactured by BASF, product name: PVP K90, 844.9 g) were put in the above fluid layer granulator, and granulation was carried out by using purified water (50 g) as a rinse.

Subsequently, the obtained granule was dried by using the granulator under the conditions of an intake air temperature of $80 \pm 5$°C and an airflow rate of $3.0 \pm 0.5$ m$^3$/min. To 100 parts by mass of the obtained granule, 19.3 parts by mass of polyacrylic acid (manufactured by the BF Goodrich Company, product name: 974P) and 1.42 parts by mass of sodium hydrogen carbonate were added, and mixed by using a high speed mixer (manufactured by Fukae Powtec Corporation, Model: FS-GS-5J) at an agitator rotation speed of $170 \pm 3$ rpm and at a chopper rotation speed of $250 \pm 3$ rpm for 15 minutes. Next, the mixture was compressed by using a roller compactor (manufactured by Freund Corporation, Model: TF-MINI), to prepare a granule for a sustained-release layer.

(3) Tabletting

The above granule for the immediate-release layer and the above granule for the sustained-release layer were put in a tabletting machine (manufactured by Hata Iron Works Co., Ltd., Model: HT-AP-38LII), and were tabletted to produce two-layer tablets of buprenorphine to be applied to mouth mucosa. The supplied amounts of both granules were adjusted such that the amount of the sustained-release layers of ten tablets became $517.8 \pm 10.2$ mg and the total amount of ten tablets became $900 \pm 17.9$ mg. In addition, the conditions of a single-layer roll, a pre-compression roll and a main compression roll were adjusted such that the tablet hardness became 4 kp or more and the tablet thickness became $1.75 \pm 0.1$ mm.

Comparative Example 2

(1) Preparation of a granule for an immediate-release layer

A buprenorphine hydrochloride crystal (100 g) was put in a sample mill (manufactured by Fuji Powdal Co., Ltd., product name: KIIW-2), and was disintegrated under the conditions of a screen aperture of 0.5 mm and a rotation speed of 12,000 rpm, to obtain about 60 g of a crystal. Next, the particle size distribution of the crystal was measured. Specifically, about 100 mg of the crystal was taken in a centrifuge tube, 10 mL of a dispersion liquid (a McIlvaine buffer solution containing 0.1 % of Triton X-100, pH9.0) was added therein, and the mixture was vigorously shaken. Immediately thereafter, the particle size distribution was measured by using a particle size analyzer (manufactured by Nikkiso Co., Ltd., Model: Microtrac FRA). As a result, the 50% cumulative diameter was 47 $\mu$m, and the 90% cumulative diameter was 232 $\mu$m.

In a rocking mixer (manufactured by Aichi Electric Co., Ltd., Model: RM-10), D-mannitol (723 g) sieved by using a sieve with an aperture of 500 $\mu$m and the above buprenorphine hydrochloride crystal (2.156 g) were put, and mixed at $60 \pm 1$ rpm for 5 minutes, and sieved by using a sieve with an aperture of 500 $\mu$m.

While purified water (1140 g) was stirred, polyvinylpyrrolidone (manufactured by ISP, product name: PVP K30, 60 g) and Food Blue No. 1 (0.0657 g) were added thereto, to prepare a solution. The above mixture powder and the solution were put in a fluid layer granulator (manufactured by Freund Corporation, Model: FLO-5B), and granulation was carried out. Subsequently, the obtained granule was dried by using the granulator under the conditions of an intake air temperature of 70 + 5°C and an airflow rate of $2.0 \pm 0.5$ m$^3$/min until the exhaust air temperature became 40°C or more. The obtained granule was sieved by using a sieve with an aperture of 500 $\mu$m.

To 100 parts by mass of the obtained granule, 1 part by mass of sodium stearate sieved by using a sieve with an aperture of 500 $\mu$m and 0.56 parts by mass of talc were added, and mixed by using a rocking mixer (manufactured by Aichi Electric Co., Ltd., Model: RM-10) at $60 \pm 1$ rpm for 3 minutes, to prepare a granule for an immediate-release layer.

(2) Preparation of a granule for a sustained-release layer

Polyvinylpyrrolidone (manufactured by BASF, product name: PVP K90, 4000 g) was put in a sample mill (manufactured by Fuji Powdal Co., Ltd., product name: KIIW-2), and was disintegrated 6 times repeatedly under the conditions of a screen aperture of 0.5 mm and a rotation speed of 12,000 rpm, to obtain about 3600 g of a crystal.

The buprenorphine hydrochloride crystal (4.312 g) disintegrated in the above Comparative Example 2 (1), the above disintegrated Polyvinylpyrrolidone (853.5 g), polyacrylic acid (manufactured by the BF Goodrich Company, product name: polyacrylic acid 974P, 165.6 g) and sodium hydrogen carbonate (12.2 g) were put in a rocking mixer (manufactured by Aichi Electric Co., Ltd., Model: RM-10), and mixed at a rotation speed of $60 \pm 1$ rpm for 15 minutes, and sieved by using a sieve with an aperture of 500 $\mu$m.

Next, the mixture was compressed by using a roller compactor (manufactured by Freund Corporation, Model: TF-MINI), and was disintegrated by using an oscillator (manufactured by Kikusui Seisakusho Ltd., Model: 35C-2M). The size of the mixture was adjusted by using a power mill (manufactured by Dalton Corporation, Model: P-02S), and then was sieved by using a sieve with an aperture of 500 $\mu$m.

(3) Tabletting

The above granule for an immediate-release layer and the above granule for a sustained-release layer were put in a tabletting machine (manufactured by Hata Iron Works Co., Ltd., Model: HT-AP-38LII), and tabletted under the same conditions as the above Comparative Example 1 (3), to produce two-layer tablets of buprenorphine to be applied to mouth mucosa.

Test Example 1: Test of determining the amount of buprenorphine derivatives

For the tablets produced in the above Comparative Examples 1 and 2, the amounts of buprenorphine derivatives immediately after the production and after the tablets were stored for 6 months in a severe environment of a temperature of 40°C and a relative humidity of 75% were measured.

Specifically, 1% by mass ammonium acetate solution and acetic acid were mixed in a volume ratio of 500 : 1, and acetonitrile for HPLC and the solution were mixed in a volume ratio of 7 : 3. One tablet of Comparative Example 1 or 2 was put in a stoppered test tube, and 1.5 mL of the above mixed solution was weighed accurately and added therein. The test tube was left at room temperature for 10 hours or longer while protected from light, and then was shaken well until the tablet sufficiently dispersed. Next, centrifuge separation was carried out at a rotation speed of 3000 rpm for 10 minutes, and the obtained clear supernatant liquid was regarded as a sample solution.

In addition, placebo powder was prepared by mixing the following ingredients.

Table 1

| | |
|---|---|
| D-mannitol specified in the Japanese Pharmacopoeia | 36.15 mg |
| Polyvinylpyrrolidone K-30 specified in the Japanese Pharmacopoeia | 1.37 mg |
| Magnesium stearate specified in the Japanese Pharmacopoeia | 0.38 mg |
| Talc specified in the Japanese Pharmacopoeia | 0.21 mg |
| Polyvinylpyrrolidone K-90 specified in the Japanese Pharmacopoeia | 42.67 mg |
| Carboxyvinyl polymer specified in the Japanese Pharmaceutical Excipients | 8.28 mg |
| Sodium hydrogen carbonate specified in the Japanese Pharmacopoeia | 0.61 mg |
| | Total 89.67 mg |

The placebo powder (0.09 g) was put in a stoppered test tube, and was subjected to the same treatment to obtain a clear supernatant liquid. The clear supernatant liquid was regarded as a placebo powder solution. Separately, buprenorphine hydrochloride (about 0.022 g) for assay was precisely weighed, and was dissolved in the above mixed solution to accurately make a solution of 100 mL.

The above solution was analyzed by HPLC, and the rate of buprenorphine derivatives $n^1$ to $n^3$ were calculated by the following mathematical formula. The results are shown in Table 2.

$$\text{Decomposed derivative amount (\%)} = (W_S / 1.078) \times (0.015 / \text{displayed amount (mg)}) \times (A^t / A^s)$$

$W_S$: amount of buprenorphine hydrochloride for assay (mg)
$A^t$: peak area of each decomposed derivative
$A^s$: peak area of buprenorphine in a standard solution

Test Example 2: Test of determining the amount of buprenorphine

For the tablets produced in the above Comparative Examples 1 and 2, the amounts of buprenorphine immediately after the production and after the tablets were stored for 6 months in a severe environment of a temperature of 40°C and a relative humidity of 75% were measured.

Specifically, twenty tablets of Comparative Example 1 or 2 were precisely weighed, and then were disintegrated into powder by using a mortar and a pestle for 30 minutes or longer. About 0.09 g of the powder was precisely weighed, and was put in a 50 mL screw cap centrifugation tube. An internal standard solution (30 mL) was accurately added therein, and was shaken well. The solution was filtered by using a membrane filter (manufactured by Nacalai Tesque, Inc., product name: Cosmonice Filter W) with an aperture of 0.45 $\mu$m, and the filtrate was regarded as a sample solution. A disintegrated material of the tablet was collected 3 times and the subsequent operations were performed once, to simultaneously prepare three sample solutions.

In addition, buprenorphine hydrochloride (about 0.032 g) for assay was precisely weighed, and was dissolved in methanol to accurately make a solution of 50 mL. The solution of 5 mL was accurately weighed, and methanol was added further to accurately make a solution of 20 mL. The solution of 2 mL was accurately taken, and the solvent was evaporated under a flow of nitrogen gas. The internal standard solution (30 mL) was accurately added to the residue, and the mixture was shaken well by using a shaker for 30 minutes. The mixture was filtered by using the above membrane filter, and the filtrate was regarded as a standard solution. Buprenorphine hydrochloride for assay was collected 3 times and the subsequent operations were carried out once, to simultaneously prepare three standard solutions.

The above sample solution and standard solution (50 $\mu$L) were analyzed by HPLC, the ratio $Q_T$ and $Q_S$ of peak heights of buprenorphine relative to the peak height of the internal standard substance were obtained, and the ratio (%) of the amount of buprenorphine in the tablet relative to the initial amount of buprenorphine was calculated from the following mathematical formula. The results are shown in Table 2.

$$\text{Buprenorphine amount (\%)} = [\text{average of } (W_S / (Q_S \times 1.078 \times \text{initial amount (mg)}))] \times [\text{average of } (Q_T \times W_W) / (W_T \times N)]$$

$W_S$: amount of buprenorphine hydrochloride for assay (mg)
$W_W$: total amount of disintegrated tablets (mg)
$W_T$: amount of disintegrated material (mg)
N: number of disintegrated tablets

Table 2

| | | Comparative Example 1 (A buprenorphine hydrochloride solution was used) | | Comparative Example 2 (Coarse buprenorphine hydrochloride was used) | |
|---|---|---|---|---|---|
| | | Initial | 6 months later | Initial | 6 months later |
| Derivative amount | n1 | 1.4% | 6.5% | 0.4% | 0.7% |
| | n2 | 1.0% | 6.4% | 0.4% | 0.6% |
| | n3 | 1.3% | 7.2% | 0.4% | 0.6% |
| | Average | 1.2% | 6.7% | 0.4% | 0.6% |
| Buprenorphine amount | Individual value | 103.1-100.7% | 96.2-95.0% | 94.6-95.2% | 93.6-93.9% |
| | Average value | 101.9% | 95.5% | 94.8% | 93.7% |

As shown in the above results, in the case of Comparative Example 1 in which buprenorphine hydrochloride was dissolved in water for producing tablets, the amount of the derivatives after the tablet was stored at 40°C for 6 months exceeds 5%. The reason is not clarified; but it is thought to be as a major reason for the result that once buprenorphine hydrochloride is dissolved as in Comparative Example 1, a matrix is formed with additives during recrystallization, resulting in that derivatives increase.

From the viewpoint of the above reason, tablets were produced directly from a crystal without making a solution as in Comparative Example 2. As a result, creation of derivatives after the tablet was stored at 40°C for 6 months was able to be suppressed to some extent. However, the amount of buprenorphine was reduced, since the coarse particles of the buprenorphine hydrochloride crystal whose 90% cumulative diameter was 232 $\mu$m might be used. Thus, the conditions for producing preparation were further studied.

Example 1

A buprenorphine hydrochloride crystal was disintegrated by using a hammer mill four times repeatedly to obtain a fine particle with a 50% cumulative diameter of 25 $\mu$m and a 90% cumulative diameter of 61 $\mu$m. A granule for the sustained-release layer was prepared similarly as in Comparative Example 2 (2), except that the buprenorphine hydrochloride crystal was used.

Example 2

The buprenorphine hydrochloride crystal used in the above Comparative Example 2 was sieved to obtain a crystal with a 50% cumulative diameter of 23 $\mu$m and a 90% cumulative diameter of 48 $\mu$m. Two-layer tablets of buprenorphine hydrochloride to be applied to mouth mucosa were prepared similarly as in Comparative Example 2, except that the buprenorphine hydrochloride crystal was used and the amount of buprenorphine hydrochloride was the half of that in Comparative Example 2.

Test Example 3: Test of determining the amount of buprenorphine

In the above Test Example 2, it was not clear whether buprenorphine was lost from the immediate-release layer or the sustained-release layer. Therefore, the amounts of buprenorphine in the granule for the immediate-release layer and in the granule for the sustained-release layer before being tabletted were measured.

Specifically, the granule for the immediate-release layer and the granule for the sustained-release layer, each containing about 0.2 to 0.6 mg of buprenorphine, were precisely weighed, and were put in centrifugation tubes, respectively. Separately, 1 mL of an imipramine hydrochloride solution as an internal standard solution was added to 120 mL of a mixed solution of acetonitrile : 2% ammonium acetate : acetic acid = 90 : 110 : 1.1, and the mixture was mixed. After the mixed solution (30 mL) was added accurately into each centrifugation tube and the tube was shaken, the mixture was filtered. Each filtrate was regarded as a sample solution. In addition, about 54 mg of buprenorphine hydrochloride for assay was precisely weighed, and methanol was added to accurately make a solution of 50 mL. The solution of 10 mL was accurately weighted, and methanol was further added to accurately make a solution of 100 mL. The solution of 2 mL was accurately taken, and was evaporated to dryness. The above mixed solution (30 mL) was added accurately thereto, the mixture was shaken and then was filtered, and the filtrate was regarded as a standard solution. The above sample solutions and standard solution (200 $\mu$L) were analyzed by HPLC, the ratios $H_T$ and $H_S$ of peak heights of buprenorphine hydrochloride relative to the peak height of the internal standard were measured, and the amount of buprenorphine hydrochloride was calculated by the following mathematical formula.

$$\text{Buprenorphine hydrochloride amount (\%)} = [\text{average of } (W_S / (Q_S \times 1.078 \times \text{initial amount (mg)}))] \times [\text{average of } (Q_T \times W_W) / (W_T \times N)]$$

$W_S$: amount of buprenorphine hydrochloride for assay (mg)
$W_W$: total amount of disintegrated tablets (mg)
$W_T$: amount of disintegrated material (mg)
N: number of disintegrated tablets

Further, the same measurement was carried out for tablets. The results are shown in Table 3. In Table 3, "CV" denotes a relative standard deviation, and is a value indicative of a degree of variation in the buprenorphine hydrochloride amount in the granule. The CV is calculated by standard deviation /average value $\times$ 100 (%). In Table 3, "upper layer" denotes "immediate-release layer", and "lower layer denotes "sustained-release layer".

Table 3

| | | Comparative Example 1 | Comparative Example 2 | | Example 1 | | Example 2 | |
|---|---|---|---|---|---|---|---|---|
| | | Aqueous solution was used | 90% cumulative diameter | Upper layer : 232μm | 90% cumulative diameter | Upper layer : 61μm | 90% cumulative diameter | Upperlayer : 48μm |
| | | | | Lower layer : 232μm | | Lower layer : 61μm | | Lower layer : 23μm |
| | | | 50% cumulative diameter | Upper layer : 47μm | 50% cumulative diameter | Upper layer : 25μm | 50% cumulative diameter | Upper layer : 48μm |
| | | | | Lower layer : 47μm | | Lower layer : 25μm | | Lower layer : 23μm |
| Granule for upper layer | Average amount | 100.4% | 102.1% | | - | | 100.1% | |
| | CV | 0.8 | 4.4 | | - | | 1.8 | |
| Granule for lower layer | Average amount | 99.0% | 95.2% | | 98.2% | | 97.1% | |
| | CV | 2.0 | 2.3 | | 0.6 | | 1.8 | |
| Tablet | Average amount | 101.9% | 94.8% | | - | | 96.1% | |

As shown in the above results, the amount of buprenorphine hydrochloride was reduced in Comparative Example 2 in which a crystal was used, as compared to Comparative Example 1 in which buprenorphine hydrochloride was dissolved in water during production of a pharmaceutical preparation. According to the result regarding Comparative Example 2, it is found that a reduction of the buprenorphine hydrochloride amount during preparation of granule occurred mainly in the sustained-release layer in case that a crystal was used for enhancing the preservation stability without dissolving buprenorphine hydrochloride in water.

From the viewpoint of the above result, the particle diameter of the buprenorphine hydrochloride crystal was reduced further in Example 1 according to the present invention. As a result, a reduction and nonuniformity of the buprenorphine hydrochloride amount in the sustained-release layer were able to be suppressed.

Further, in Example 2 for confirming the reproducibility, tablets whose buprenorphine hydrochloride amount was reduced by half to 0.15 mg were produced as a severer condition. As a result, even in the 0.15 mg tablet in which a reduction and nonuniformity of the buprenorphine hydrochloride amount were likely to occur, such reduction and nonuniformity were able to be suppressed by adjusting the 90% cumulative diameter of the used material powder.

Thus, it was demonstrated that a highly stable buprenorphine pharmaceutical preparation to be applied to mouth mucosa can be produced while the buprenorphine hydrochloride amount is maintained by the process of the present invention.

Test Example 4: Test of determining the amount of buprenorphine

Based on the above Test Example 3, buprenorphine hydrochloride crystals with various particle diameters were prepared, and the similar test was carried out. The results are shown in Table 4. Further, a relation between the 90% cumulative diameter of a buprenorphine hydrochloride crystal and the CV of an immediate-release layer or a sustained-release layer is shown in Fig. 1 or 2. In Table 4, "upper layer" denotes "immediate-release layer", and "lower layer" denotes "sustained-release layer".

Table 4

| Pharmaceutical preparation No. | | 1 | 2 | 3 | 4 | 5 | 6 | 7 | 8 | 9 | 10 |
|---|---|---|---|---|---|---|---|---|---|---|---|
| Particle diameter of active pharmaceutical ingredient | 90% cumulative diameter ($\mu$m) | 466 | 315 | 232 | 185 | 156 | 110 | 84 | 67 | 60 | 45 |
| | 50% cumulative diameter ($\mu$m) | 200 | 121 | 47 | 52 | 50 | 48 | 42 | 33 | 28 | 22 |
| Granule for upper layer | Average amount | 96.4 | 95.5 | 102.0 | 96.5 | 98.1 | 99.6 | 97.5 | 95.1 | 102.2 | 98.2 |
| | CV | 7.5 | 6.7 | 4.3 | 2.4 | 1.7 | 1.7 | 1.9 | 1.2 | 2.0 | 1.0 |
| Granule for lower layer | Average amount | 88.7 | 91.0 | 95.2 | 94.3 | 96.9 | 97.4 | 100.8 | 102.0 | 100.1 | 99.5 |
| | CV | 9.4 | 8.0 | 2.3 | 2.8 | 1.6 | 1.7 | 0.5 | 1.0 | 0.5 | 1.2 |

As shown in Table 4 and Figs. 1 and 2, the CV starts to rise when the 90% cumulative diameter of the buprenorphine hydrochloride crystal exceeds 200 $\mu$m in the immediate-release layer, and the CV rises sharply when the 90% cumulative diameter exceeds 250 $\mu$m in the sustained-release layer. From the result, it is found that for equalizing the buprenorphine hydrochloride amount of tablet, it is important that the 90% cumulative diameter of the buprenorphine hydrochloride crystal for the immediate-release layer is adjusted to 200 $\mu$m or less and the 90% cumulative diameter of the buprenorphine hydrochloride crystal for the sustained-release layer is adjusted to 250 $\mu$m or less. Further, it was found from the result that a smaller 50% cumulative diameter is better for equalizing the buprenorphine hydrochloride amounts; and hence, it is thought that the 50% cumulative diameter is preferably 100 $\mu$m or less. However, there was the case where the CV was high even when the 50% cumulative diameter was 50 $\mu$m or less. Thus, it was found that it is more important to adjust the 90% cumulative diameter of a crystal than the 50% cumulative diameter of the crystal for equalizing the buprenorphine hydrochloride amount of pharmaceutical preparation.

**EP 2 123 275 A1**

INDUSTRIAL APPLICABILITY

According to the production process of the present invention, a buprenorphine pharmaceutical preparation to be applied to mouth mucosa having excellent stability and uniform amount of active ingredient therein can be produced. Thus, the present invention is industrially extremely useful for the production of a buprenorphine pharmaceutical preparation to be applied to mouth mucosa, which is used for the therapy of postherpetic neuralgia and the like.

**Claims**

1. A process for producing a buprenorphine pharmaceutical preparation to be applied to mouth mucosa, comprising steps of:

   preparing a granule for an immediate-release layer containing a buprenorphine hydrochloride crystal having 90% cumulative diameter of 200 $\mu$m or less;
   preparing a granule for a sustained-release layer containing a buprenorphine hydrochloride crystal having 90% cumulative diameter of 250 $\mu$m or less; and
   tabletting the granule for the immediate-release layer and the granule for the sustained-release layer into a two-layer tablet.

2. The process according to claim 1, wherein the 50% cumulative diameters of the buprenorphine hydrochloride crystals of the granule for the immediate-release layer and the granule for the sustained-release layer are 60 $\mu$m or less.

3. The process according to claim 1 or 2, wherein the granule is subjected to disintegration and size-regulation in the step of preparing the granule for the sustained-release layer.

4. The process according to any one of claims 1 to 3, wherein the granule for the immediate-release layer contains D-mannitol and/or talc.

5. The process according to any one of claims 1 to 4, wherein the granule for the sustained-release layer contains polyvinylpyrrolidone or a pharmaceutically acceptable salt thereof, polyacrylic acid or a pharmaceutically acceptable salt thereof, and sodium hydrogen carbonate.

Fig. 1

Fig. 2

## INTERNATIONAL SEARCH REPORT

| | International application No. |
|---|---|
| | PCT/JP2008/054794 |

A. CLASSIFICATION OF SUBJECT MATTER
*A61K31/485*(2006.01)i, *A61K9/22*(2006.01)i, *A61K9/70*(2006.01)i, *A61K47/26* (2006.01)i, *A61K47/32*(2006.01)i, *A61P25/04*(2006.01)i

According to International Patent Classification (IPC) or to both national classification and IPC

B. FIELDS SEARCHED

Minimum documentation searched (classification system followed by classification symbols)
A61K31/485, A61K9/22, A61K9/70, A61K47/26, A61K47/32, A61P25/04

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 8-291070 A (Toyobo Co., Ltd.), 05 November, 1996 (05.11.96), Examples 3 to 6; column 3, lines 4 to 9; column 6, lines 13 to 22 (Family: none) | 1-5 |
| Y | Edited by Mitsuo MATSUMOTO et al., Yakuzaigaku Manual, issued by Nanzando Co., Ltd., 20 March, 1989 (20.03.89), 1st edition, pages 80, 88 to 89 | 1-5 |
| Y | Edited by Sadasuke OKANO et al., Shin · Yakuzaigaku Soron, issued by Nankodo Co., Ltd., 10 April, 1987 (10.04.87), revised edition No.3, pages 109, 123 to 124, 138 to 139, 265 | 1-5 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 April, 2008 (24.04.08) | 13 May, 2008 (13.05.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**EP 2 123 275 A1**

<table>
<tr><td colspan="2"><strong>INTERNATIONAL SEARCH REPORT</strong></td><td>International application No.<br>PCT/JP2008/054794</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 00/12063 A1  (Teijin Ltd.),<br>09 March, 2000 (09.03.00),<br>Page 16, lines 6 to 7<br>& EP 1108423 A1        & JP 2000-567183 A<br>& US 6835389 B1        & EP 1108423 B1 | 1-5 |
| A | JP 8-333243 A  (Nippon Kayaku Co., Ltd.),<br>17 December, 1996 (17.12.96),<br>(Family: none) | 1-5 |

Form PCT/ISA/210 (continuation of second sheet) (April 2007)

**15**

| INTERNATIONAL SEARCH REPORT | International application No. |
|---|---|
| | PCT/JP2008/054794 |

<Concerning the scope of search>
    The wording "the step of preparing granules for rapid release layer containing buprenorphine hydrochloride crystals whose 90% cumulative diameter is 200μm or below" set forth in claim 1 has two interpretations, namely, the case wherein the 90% cumulative diameter of buprenorphine hydrochloride crystals is 200μm or below and the case wherein the 90% cumulative diameter of granules for rapid release layer is 200μm or below. Thus, the wording is unclear.
    In this international search report, however, the case wherein the 90% cumulative diameter of buprenorphine hydrochloride crystals is 200μm or below is accepted, because the description does not include any explanation about the size of the granules. Although the description discloses the step of preparing granules for rapid release layer by using buprenorphine hydrochloride crystals having a 90% cumulative diameter of 200μm or below, the particle diameter of buprenorphine hydrochloride crystals in the obtained granules is not disclosed. Further, it is unclear how the particle diameter of buprenorphine hydrochloride crystals in the granules is controlled or determined.
    Thus, it cannot be considered that "the step of preparing granules for rapid release layer containing buprenorphine hydrochloride crystals whose 90% cumulative diameter is 200μm or below" is specifically disclosed in the description and supported by the description.
    The same applies to "the step of preparing granules for sustained release layer containing buprenorphine hydrochloride crystals whose 90% cumulative diameter is 250μm or below".
    As to the process of claim 2 wherein "the 50% cumulative diameters of buprenorphine hydrochloride crystals in the granules for the rapid release layer and the granules for the sustained release layer are 60μm or below", the preparation of granules by using buprenorphine hydrochloride crystals whose 50% cumulative diameter is 60μm or below is disclosed in the description, but the particle diameter of buprenorphine hydrochloride crystals in the obtained granules is not disclosed. Further, it is unclear how the particle diameter of buprenorphine hydrochloride crystals in the granules is controlled or determined.
    Thus, it cannot be considered that the process wherein "the 50% cumulative diameters of buprenorphine hydrochloride crystals in the granules for rapid release layer and the granules for sustained release layer are 60μm or below" is specifically disclosed in the description and supported by the description.
    Therefore, claims 1-5 and the description fail to comply with the prescribed requirements to such an extent that a meaningful search could not be carried out.
    Consequently, search has been made with priority given to matter supported by the description and disclosed in the description.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8291070 A

- JP 2001506640 T